# DEMANDE DE BREVET EUROPEEN

(11) **EP 2 133 333 A1**
(43) Date de publication de la demande: **16.12.2009**
(21) Numéro de dépôt: 09290432.5
(22) Date de dépôt: 11.06.2009
(51) Int. Cl.: C07D 209/52, A61K 31/403, A61P 25/00, A61P 3/04

(54) **Noveaux dérivés azabicycliques, leur procédé de préparation et les compositions pharmaceutiques qui les contiennent**

(30) Priorité: 13.06.2008 FR 0803297
(71) Demandeur: Les Laboratoires Servier, 92284 Suresnes Cedex (FR)
(72) Inventeur: Casara, Patrick, 78670 Villennes sur Seine (FR); Chollet, Anne-Marie, 78230 Le Pecq (FR); Dhainaut, Alain, 78400 Chatou (FR); Lestage, Pierre, 78170 La Celle Saint Cloud (FR); Panayi, Fany, 75018 Paris (FR); Roger, Anita, 78620 L'Etang-la-Ville (FR)

(57) **Abrégé**

Composés de formule (I) : 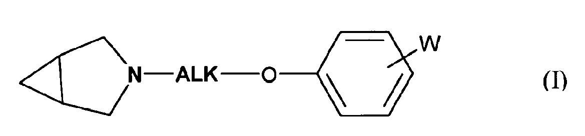 dans laquelle :
◆ Alk représente une chaîne alkylène,
◆ W représente un groupement choisi parmi 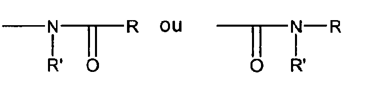 où R et R' sont tels que définis dans la description.

Médicament.

## Description

La présente invention concerne de nouveaux dérivés azabicycliques, leur procédé de préparation et les compositions pharmaceutiques qui les contiennent.

Les composés de la présente invention sont particulièrement intéressants d'un point de vue pharmacologique pour leur interaction avec les systèmes histaminergiques centraux *in vivo.*

Le vieillissement de la population, du fait de l'augmentation de l'espérance de vie à la naissance, a entraîné parallèlement un large accroissement de l'incidence des neuropathologies liées à l'âge, et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence.

Au niveau du système nerveux central, des études neuropharmacologiques ont montré que l'histamine *via* les systèmes histaminergiques centraux jouait un rôle de neurotransmetteur ou neuromodulateur en situations physiologiques ou physio-pathologiques (Pell et Green, Annu. Rev. Neurosci., 1986, 9, 209-254; Schwartz et al., Physiol. Rev., 1991, 71, 1-51). Ainsi, il a été montré que l'histamine intervenait dans divers processus physiologiques et comportementaux tels que la thermorégulation, la régulation neuro-endocrinienne, le rythme circadien, les états cataleptiques, la motricité, l'agressivité, le comportement alimentaire, l'apprentissage et la mémorisation, ainsi que la plasticité synaptique (Hass et al., Histaminergic neurones : morphology and function, Boca Raton, FL : CRC Press, 1991, pp. 196-208 ; Brown et al., Prog. Neurobiology, 2001, 63, 637-672).

Des études, réalisées chez l'animal, ont montré que l'augmentation des taux endogènes extra-synaptiques d'histamine permettait de promouvoir les états de vigilance, les processus d'apprentissage et de mémoire et de réguler la prise alimentaire (Brown et al., Prog. Neurobiol., 2000, 63, 637-672; Passani et al., Neurosci. Biobehav. Rev., 2000, 24, 107-113). En conséquence, les indications thérapeutiques potentielles pour des composés capables d'augmenter le turn-over ou la libération d'histamine au niveau central sont le traitement des déficits cognitifs associés au vieillissement cérébral, aux maladies neurodégénératives aigues et chroniques, à la schizophrénie, ainsi que le traitement des troubles de l'humeur, de la schizophrénie, des troubles du sommeil et du rythme veille-sommeil, du syndrome d'hyperactivité avec déficits attentionnels. Par ailleurs, des travaux ont montré qu'une injection d'histamine au niveau des noyaux centraux hypothalamiques impliqués dans la régulation de la satiété atténue l'alimentation chez le rat. De plus, un hypofonctionnement de la transmission histaminergique a été mis en évidence chez des rats génétiquement obèses (Machidori et al., Brain Research, 1992, 590, 180-186). En conséquence, les troubles du comportement alimentaire et l'obésité sont également des indications thérapeutiques potentielles pour les composés de la présente invention.

La présente invention concerne de nouveaux dérivés azabicycliques qui se distinguent des composés exemplifiés dans la demande WO2005/089747 par la présence d'un noyau 3-azabicyclo[3,1,0]hexane.

De manière surprenante, cette différence structurale par rapport aux composés de la demande WO2005/089747 confère aux composés de l'invention non seulement des propriétés procognitives remarquables, mais également de puissantes propriétés éveillantes, anti-sédatives, anti-hypnotiques et antidépressives.

Au niveau neurologique, cette combinaison d'activités ouvre la voie non seulement à de nouveaux traitements des troubles cognitifs liés au vieillissement cérébral, aux maladies neurodégénératives ou aux traumatismes crâniens, mais également au traitement des troubles psychocomportementaux associés à ces pathologies tels que les troubles du sommeil, l'apathie et/ou les états dépressifs. Par ailleurs, le profil pharmacologique des composés de l'invention permet aussi d'envisager de nouveaux traitements dans le domaine psychiatrique, pour la schizophrénie, les troubles de l'humeur ou du sommeil par exemple.

La présente invention concerne plus particulièrement les composés de formule (I) : 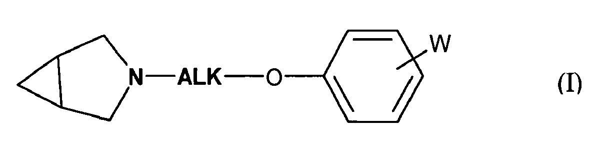 dans laquelle :
◆ Alk représente une chaîne alkylène,
◆ W représente un groupement choisi parmi 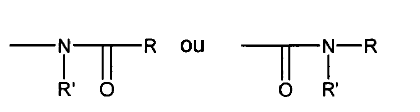 où R et R' représentent indépendamment l'un de l'autre un hydrogène ou un groupement alkyle (C₁-C₆) linéaire ou ramifié éventuellement substitué par un halogène, un groupement hydroxy ou un groupement alkoxy,
étant entendu que :
- le terme alkylène désigne un radical bivalent, linéaire ou ramifié, contenant de 2 à 6 atomes de carbone,
- le terme alkoxy désigne un groupement alkyle-oxy dont la chaîne alkyle, linéaire ou ramifiée, contient de 1 à 6 atomes de carbone,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc.

Parmi les bases pharmaceutiquement acceptables, on peut citer à titre non limitatif l'hydroxyde de sodium, l'hydroxyde de potassium, la triéthylamine, la tertbutylamine, etc.

Les composés de formule (I) préférés sont ceux pour lesquels le groupement W est situé en position para.

De manière préférentielle, Alk représente un groupe éthylène, propylène ou butylène, et plus préférentiellement encore un groupe propylène.

Un aspect particulier de l'invention concerne les composés de formule (I) pour lesquels W représente un groupement -CO-NH₂, -NH-CO-CH₃, -N(CH₃)-CO-CH₃ ou -NH-CO-CH₂-OCH₃.

Encore plus particulièrement, l'invention concerne les composés de formule (I) qui sont :
- le *N*-(4-{3-[(1*R*,5*S*)-3-azabicyclo[3.1.0]hex-3-yl]propoxy}phényl)-*N-*méthylacétamide,
- le *N*-(4-{2-[(1*R*,5*S*)-3-azabicyclo[3.1.0]hex-3-yl]éthoxy}phényl)acétamide,
- le *N*-(4-{2-[(1*R*,5*S*)-3-azabicyclo[3.1.0]hex-3-yl]éthoxy}phényl)-2-méthoxyacétamide,
- le 4-{3-[(1*R*,5*S*)-3-azabicyclo[3.1.0]hex-3-yl]propoxy}benzamide,
- le 4-{2-[(1*R*,5*S*)-3-azabicyclo[3.1.0]hex-3-yl]éthoxy}benzamide,
- le *N*-(4-{3-[(1*R*,5*S*)-3-azabicyclo[3.1.0]hex-3-yl]propoxy}phényl)-2-méthoxyacétamide,
- le *N*-(4-{3-[(1*R*,5*S*)-3-azabicyclo[3.1.0]hex-3-yl]propoxy}phényl)acétamide,
et leurs sels d'adition à un acide ou à une base pharmaceutiquement acceptables.

Parmi les sels d'adition à un acide pharmaceutiquement acceptable, on préfère plus particulièrement les chlorhydrate, oxalate et citrate.

Le chlorhydrate de *N*-(4-{3-[(1*R*,5*S*)-3-azabicyclo[3.1.0]hex-3-yl]propoxy}phényl)-*N-*méthylacétamide est préféré.

Le chlorhydrate de 4-{3-[(1*R*,5*S*)-3-azabicyclo[3.1.0]hex-3-yl]propoxy}benzamide, l'oxalate de 4-{3-[(1*R*,5*S*)-3-azabicyclo[3.1.0]hex-3-yl]propoxy}benzamide et le citrate de 4-{3-[(1*R*,5*S*)-3-azabicyclo[3.1.0]hex-3-yl]propoxy}benzamide sont plus particulièrement encore préférés.

L'invention s'étend également au procédé de préparation des composés de formule (I) **caractérisé en ce que** l'on utilise comme produit de départ le composé de formule (II) : 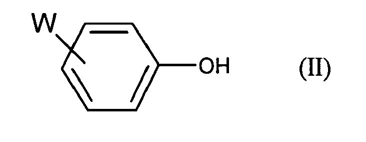 dans laquelle W est tel que défini dans la formule (I),
composé de formule (II) sur lequel on condense en milieu basique le composé de formule (III) :

Br-ALK-Cl (III)

pour obtenir le composé de formule (IV) : 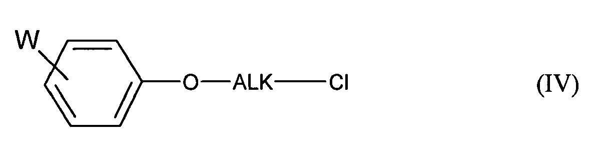 dans laquelle W et Alk sont tels que définis précédemment,
sur lequel on condense le composé de formule (V) : 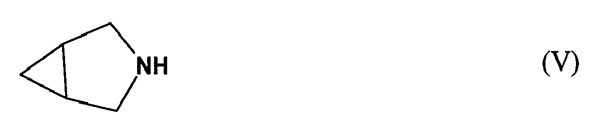 pour conduire au composé de formule (I) tel que défini précédemment : 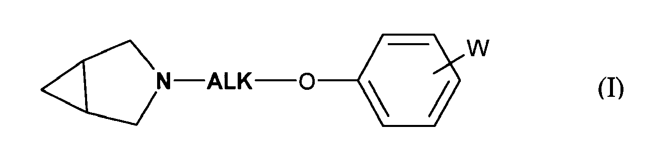 qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

Les composés de formules (II), (III) et (V) sont soit commerciaux, soit accessibles à l'homme du métier par des réactions chimiques classiques et décrites dans la littérature.

Alternativement, les composés de formule (VI) : 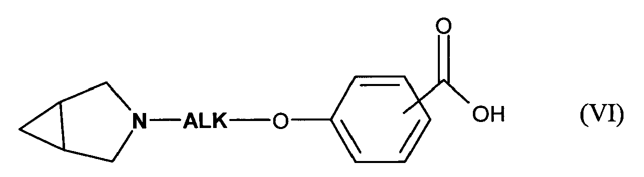 dans lesquels le groupement Alk est tel que défini précédemment,
peuvent être utilisés en tant qu'intermédiaires de synthèse des composés de formule (I) pour lesquels W=-CONRR' par couplage avec une amine de formule NHRR', où R et R' ont la même signification que dans la formule (I).

Pour la suite, les composés de formule (I) pour lesquels W=-CONRR' seront appelés composés de formule (I/a).

De même, les composés de formule (VII) : 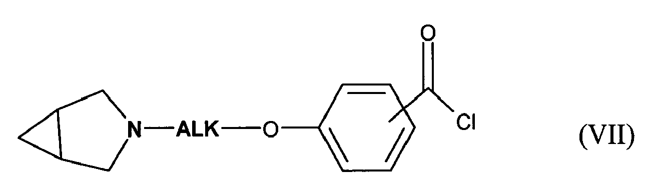 dans lesquels le groupement Alk est tel que défini précédemment,
peuvent être utilisés en tant qu'intermédiaires de synthèse des composés de formule (I/a) par couplage avec une amine de formule NHRR', où R et R' ont la même signification que dans la formule (I).

De plus, les composés de formule (I/a) peuvent aussi être obtenus en utilisant les composés de formule (VIII) via l'acide carboxylique (VI) et le chlorure d'acyle (VII) correspondant présentés au-dessus : 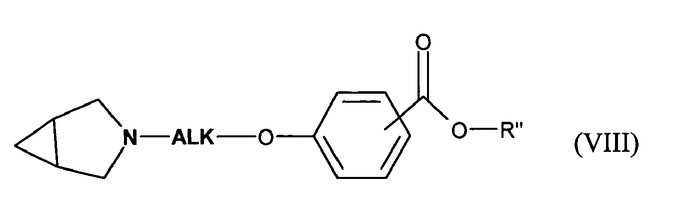 dans lesquels le groupement Alk est tel que défini précédemment et R" représente un groupement alkyle (C₁-C₆) linéaire ou ramifié.

Une autre alternative consiste à utiliser les composés de formule (VIII) en présence d'une amine de formule NHRR' pour conduire directement aux composés de formule (I/a).

Enfin, il est également possible d'obtenir les composés de formule (I/a) en hydrolysant des composés de formule (IX) en milieu basique : 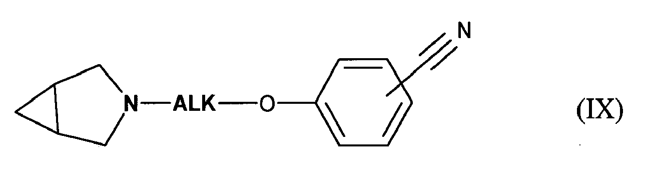 dans lesquels le groupement Alk est tel que défini précédemment.

L'étude pharmacologique des composés de formule (I) a montré qu'ils possédaient des propriétés procognitives en facilitant les processus de mémorisation et d'apprentissage, des propriétés éveillantes, anti-sédatives, anti-hypnotiques ainsi que des propriétés antidépressives. Par ailleurs, les composés de formule (IX) possèdent également des propriétés procognitives.

Au niveau neurologique, les composés selon l'invention peuvent être utiles dans le traitement des troubles cognitifs liés au vieillissement cérébral ou aux maladies neurodégénératives telles que la maladie d'Alzheimer, la maladie de Parkinson, la maladie de Pick, les démences à corps de Lewy, les démences frontales et sous-corticales, les démences frontotemporales, les démences vasculaires, la maladie de Huntington, la sclérose en plaques, dans de nouveaux traitements des troubles cognitifs liés aux traumatismes crâniens, mais également dans le traitement des troubles psychocomportementaux associés à ces pathologies tels que les troubles du sommeil, l'apathie et la dépression. Les troubles du sommeil associés à la maladie d'Alzheimer et à la maladie de Parkinson, tels que les hypersomnolences diurnes, sont particulièrement visés.

Au niveau psychiatrique, ces composés peuvent être utiles dans le traitement des troubles de l'humeur, et plus particulièrement le traitement des états dépressifs, de la schizophrénie et des troubles cognitifs qui lui sont associés, ainsi que dans le traitement des troubles du sommeil, du rythme veille-sommeil et du syndrome d'hyperactivité avec déficits attentionnels (ADHD). Parmi les troubles du sommeil, on peut citer plus particulièrement la narcolepsie, les hypersomnies survenant lors du syndrome d'apnée obstructive du sommeil ou du syndrome d'hyperactivité avec déficits attentionnels, ainsi que les somnolences diurnes.

La présente invention a également pour objet les compositions pharmaceutiques contenant au moins un composé de formule (I) seul ou en combinaison avec un ou plusieurs excipients pharmaceutiquement acceptables.

Dans les compositions pharmaceutiques selon l'invention, la fraction massique en principe actif (masse du principe actif sur la masse totale de la composition) est comprise entre 1 et 50%.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,05 mg et 500 mg par 24 heures pour un traitement en 1 à 3 prises par jour.

Les exemples suivants illustrent l'invention et ne la limitent en aucune façon. Les structures des composés, décrits dans les exemples, ont été déterminées selon les techniques spectrophotométriques usuelles (infrarouge, RMN, spectrométrie de masse...).

A titre d'information, tous les composés ci-dessous présentent une stéréochimie de type méso.

### Exemple 1, voie de synthèse A : Chlorhydrate de 4-{3-[(1R,5S)-3-azabicyclo[3.1.0]hex-3-yl]propoxy} benzamide

### Stade 1 : 4-(3-Chloropropoxy)benzamide

Un mélange constitué de 0,004 mole de 4-hydroxybenzamide, de 0,004 mole de 1-bromo-3-chloropropane et de 0,006 mole de carbonate de césium dans 10ml d'acétonitrile est chauffé au reflux pendant 5 heures.

### Stade 2 : 4-{3-[(1R,5S)-3-Azabicyclo[3.1.0]hex-3-yl]propoxy}benzamide

Dans le milieu réactionnel du stade 1 à température ambiante sont ajoutées 0,004 mole de (1*R*,5*S*)-3-azabicyclo[3.1.0]hexane et 0,002 mole d'iodure de sodium. Le chauffage au reflux est ensuite repris pendant 16 heures. Le précipité est filtré, rincé avec de l'acétonitrile. Le filtrat est concentré à sec. Le résidu est repris dans du dichlorométhane. Cette solution est extraite avec de la soude, puis de l'eau, avant d'être séchée sur sulfate de magnésium et concentrée à sec. Le résidu est purifié par technique de chromatographie préparative sur phase Lichroprep RP-18.

### Spectre de masse : [M+H]⁺ m/z théorique = 243,1510 ; mlz expérimental = 243,1497

### Stade 3 : Chlorhydrate de 4-{3-[(1R,5S)-3-azabicyclo[3.1.0]hex-3-yl]propoxy}benzamide

Le produit obtenu au stade 2 est dissous dans 10 ml d'éthanol auxquels sont ajoutés 2 ml d'éther chlorhydrique 2 N. Le produit cristallisé est filtré, rincé avec de l'éthanol et séché sous vide.

### Microanalyse élémentaire :

| | *% C* | *% H* | *% N* | *%Cl* | *%Cl⁻* |
|---|---|---|---|---|---|
| *Calculé* | *60,70* | *7,13* | *9,44* | *11,95* | *11,95* |
| *Trouvé* | *60,74* | *7, 04* | *9,31* | *11,89* | *11,87* |

### Exemple 1, voie B: Chlorhydrate de 4-{3-[(1R,5S)-3-azabicyclo[3.1.0]hex-3-yl]propoxy}benzamide

### Stade 1 : 4-{3-[(1R,5S)-3-Azabicyclo[3.1.0]hex-3-yl]propoxy}benzonitrile

Le procédé expérimental est identique aux stades 1 et 2 de l'Exemple 1, voie de synthèse A, en remplaçant au stade 1 le 4-hydroxybenzamide par le 4-hydroxybenzonitrile.

### Spectre de masse : [M+H]⁺ mlz théorique = 243,1510 ; mlz expérimental = 243,1497

### Stade 2 : Chlorhydrate de 4-{3-[(1R,5S)-3-azabicyclo[3.1.0]hex-3-yl]propoxy}benzamide

Le composé obtenu au stade précédent (2,2g) est mis en solution dans 90 ml d'éthanol et chauffé à reflux en présence de 5,1g de KOH pendant 18h. Le milieu est versé dans 90 ml d'eau puis concentré à mi-volume sous vide. Le solide obtenu est filtré, rincé avec de l'éther isopropylique, puis séché. Le chlorhydrate est préparé suivant le mode opératoire du stade 3 de l'Exemple 1, voie de synthèse A.

### Microanalyse élémentaire :

| | *% C* | *% H* | *% N* | *%Cl* | *%Cl⁻* |
|---|---|---|---|---|---|
| *Calculé* | *60,70* | *7,13* | *9,44* | *11,95* | *11,95* |
| *Trouvé* | *60,36* | *7,11* | *9,11* | *11,36* | *11,93* |

### Exemple 1, voie C : Chlorhydrate de 4-{3-[(1R,5S)-3-azabicyclo[3.1.0]hex-3-yl]propoxy}benzamide

### Stade 1 : 4-{3-[(1R,5S)-3-Azabicyclo[3.1.0]hex-3-yl]propoxy}benzoate de méthyle

Le procédé expérimental est identique aux stades 1 et 2 de l'Exemple 1, voie de synthèse A, en remplaçant au stade 1 le 4-hydroxybenzamide par le 4-hydroxybenzoate de méthyle.

### Stade 2 : Acide 4-{3-[(1R,5S)-3-azabicyclo[3.1.0]hex-3-yl]propoxy}benzoïque

Un mélange de 3,5 g du composé du stade 1, de 12,7 ml de soude 2 N et de 8 ml de méthanol est chauffé à reflux pendant une heure. Dans le milieu réactionnel refroidi au bain de glace sont ajoutés 12,7 ml d'HCl 2 N. Le précipité est lavé à l'eau et séché sous vide.

### Microanalyse élémentaire :

| | *% C* | *% H* | *% N* |
|---|---|---|---|
| *Calculé* | *68,94* | *7,33* | *5,36* |
| *Trouvé* | *68,74* | *7,32* | *5,42* |

### Stade 3 : Chlorhydrate de chlorure de 4-{3-[(1R,5S)-3-azabicyclo[3.1.0]hex-3-yl]propoxy} benzoyle

Un mélange de 1,8 g de produit décrit au stade 2 et de 20 ml de chlorure de thionyle est chauffé à reflux pendant 2 heures. Le milieu réactionnel est concentré sous vide, coévaporé deux fois avec du toluène. Le résidu solide est homogénéisé dans l'éther éthylique, filtré et séché sous vide.

### Stade 4 : Chlorhydrate de 4-{3-[(1R,5S)-3-azabicyclo[3.1.0]hex-3-yl]propoxy}benzamide

Dans une solution de 1 g de produit décrit au stade 3 dans le dichlorométhane à 0°C, sont ajoutés goutte à goutte 2 ml de méthanol ammoniacal 2 N. Le mélange est ensuite agité 1 heure à température ambiante, lavé avec un solution de soude 2 N, puis de l'eau. La phase organique est séchée sur sulfate de magnésium et concentrée. L'huile résiduelle est dissoute dans 10 ml d'éthanol auxquels sont ajoutés 2 ml d'éther chlohydrique 2 N. Le produit cristallisé est filtré, rincé avec de l'éthanol et séché sous vide.

### Microanalyse élémentaire :

| | *% C* | *% H* | *% N* | *% Cl* |
|---|---|---|---|---|
| *Calculé* | *60,70* | *7,13* | *9,44* | *11,95* |
| *Trouvé* | *60,74* | *7,04* | *9,31* | *11,89* |

### Exemple 2 : 4-{2-[(1R,5S)-3-Azabicyclo[3.1.0]hex-3-yl]éthoxy}benzamide

Le procédé expérimental est identique aux stades 1 et 2 de l'Exemple 1, voie de synthèse A, en remplaçant au stade 1 le 1-bromo-3-chloropropane par le 1-bromo-2-chloroéthane.

### Microanalyse élémentaire :

| | *% C* | *% H* | *% N* |
|---|---|---|---|
| *Calculé* | *68,27* | *7,37* | *11,37* |
| *Trouvé* | *67,07* | *7,12* | *11,15* |

### Exemple 3 : 4-14-[(1R,5S)-3-Azabicyclo[3.1.0]hex-3-yl]butoxy}benzamide

Le procédé expérimental est identique aux stades 1 et 2 de l'Exemple 1, voie de synthèse A, en remplaçant au stade 1 le 1-bromo-3-chloropropane par le 1-bromo-4-chlorobutane.

### Microanalyse élémentaire :

| | *% C* | *% H* | *% N* |
|---|---|---|---|
| *Calculé* | *70,04* | *8,08* | *10,21* |
| *Trouvé* | *69,96* | *8,07* | *9,91* |

### Exemple 4 : N-(4-{3-[(1R,5S)-3-Azabicyclo[3.1.0]hex-3-yl]propoxy}phényl)acétamide

Le procédé expérimental est identique aux stades 1 et 2 de l'Exemple 1 en remplaçant au stade 1 le 4-hydroxybenzamide par le *N*-(4-hydroxyphényl)acétamide.

### Microanalyse élémentaire :

| | *% C* | *% H* | *% N* |
|---|---|---|---|
| *Calculé* | *70,04* | *8,08* | *10,21* |
| *Trouvé* | *69,77* | *8,02* | *10,00* |

### Exemple 5 : N-(4-{2-[(1R,5S)-3-Azabicyclo[3.1.0]hex-3-yl]éthoxy}phényl)acétamide

Le procédé expérimental est identique aux stades 1 et 2 de l'Exemple 2 en remplaçant au stade 1 le 4-hydroxybenzamide par le *N*-(4-hydroxyphényl)acétamide.

### Microanalyse élémentaire :

| | *% C* | *% H* | *% N* |
|---|---|---|---|
| *Calculé* | *69,21* | *7,74* | *10,76* |
| *Trouvé* | *68,86* | *7,66* | *10,55* |

### Exemple 6 : N-(4-{4-[(1R,5S)-3-Azabicyclo[3.1.0]hex-3-yl]butoxy}phényl)acétamide

Le procédé expérimental est identique aux stades 1 et 2 de l'Exemple 3 en remplaçant au stade 1 le 4-hydroxybenzamide par le *N*-(4-hydroxyphényl)acétamide.

### Microanalyse élémentaire :

| | *% C* | *% H* | *% N* |
|---|---|---|---|
| *Calculé* | *70,80* | *8,39* | *9,71* |
| *Trouvé* | *70,44* | *8,35* | *9,14* |

### Exemple 7 : 4-{3-[(1R,5S)-3-Azabicyclo[3.1.0]hex-3-yl]propoxy}-N,N-diméthyl benzamide

Le procédé expérimental est identique aux stades 1 et 2 de l'Exemple 1, voie de synthèse A, en remplaçant au stade 1 le 4-hydroxybenzamide par le 4-hydroxy-*N,N-*diméthylbenzamide.

### Microanalyse élémentaire :

| | *% C* | *% H* | *% N* |
|---|---|---|---|
| *Calculé* | *70,80* | *8,39* | *9,71* |
| *Trouvé* | *70,82* | *8,32* | *9, 60* |

### Exemple 8 : Oxalate de 4-{4-[(1R,5S)-3-azabicyclo[3.1.0]hex-3-yl]butoxy}-N,N-diméthylbenzamide

Le procédé expérimental est identique aux stades 1 et 2 de l'Exemple 3 en remplaçant au stade 1 le 4-hydroxybenzamide par le 4-hydroxy-*N,N*-diméthylbenzamide. Puis, on ajoute 0,32 g d'acide oxalique à 0,38 g du composé ainsi obtenu dans 6 ml d'éthanol. Le produit obtenu est filtré, rincé à l'éther éthylique puis séché sous vide.

### Microanalyse élémentaire :

| | *% C* | *% H* | *% N* |
|---|---|---|---|
| *Calculé* | *61,21* | *7,19* | *7,14* |
| *Trouvé* | *60,13* | *6,93* | *6,71* |

### Exemple 9 : Chlorhydrate de 4-{3-[(1R,5S)-3-azabicyclo[3.1.0]hex-3-yl]propoxy}-N,N-diéthylbenzamide

Le procédé expérimental est identique à celui de l'Exemple 1, voie de synthèse A, en remplaçant au stade 1 le 4-hydroxybenzamide par le 4-hydroxy-*N,N*-diéthylbenzamide.

### Microanalyse élémentaire :

| | *% C* | *% H* | *% N* | *% Cl* | *% Cl⁻* |
|---|---|---|---|---|---|
| *Calculé* | *64,67* | *8,28* | *7,94* | *10,05* | *10,05* |
| *Trouvé* | *64,92* | *7,59* | *8,24* | *12,57* | *10,78* |

### Exemple 10 : 4-{4-[(1R,5S)-3-Azabicyclo[3.1.0]hex-3-yl]butoxy}-N,N-diéthyl benzamide

Le procédé expérimental est identique aux stades 1 et 2 de l'Exemple 3 en remplaçant au stade 1 le 4-hydroxybenzamide par le 4-hydroxy-*N,N*-diéthylbenzamide.

### Microanalyse élémentaire :

| | *% C* | *% H* | *% N* |
|---|---|---|---|
| *Calculé* | *72,69* | *9,15* | *8,48* |
| *Trouvé* | *72,45* | *9,02* | *8,33* |

### Exemple 11 : 4-{3-[(1R,5S)-3-Azabicyclo[3.1.0]hex-3-yl]propoxy}-N-méthylbenzamide

Le procédé expérimental est identique aux stades 1 et 2 de l'Exemple 1, voie de synthèse A, en remplaçant au stade 1 le 4-hydroxybenzamide par le 4-hydroxy-*N*-méthylbenzamide.

### Microanalyse élémentaire :

| | *% C* | *% H* | *% N* |
|---|---|---|---|
| *Calculé* | *70, 04* | *8, 08* | *10, 21* |
| *Trouvé* | *69,93* | *8, 00* | *10, 09* |

### Exemple 12 : 4-{4-[(1R,5S)-3-Azabicyclo[3.1.0]hex-3-yl]butoxy}-N-méthylbenzamide

Le procédé expérimental est identique aux stades 1 et 2 de l'Exemple 3 en remplaçant au stade 1 le 4-hydroxybenzamide par le 4-hydroxy-*N*-méthylbenzamide.

### Microanalyse élémentaire :

| | *% C* | *% H* | *% N* |
|---|---|---|---|
| *Calculé* | *70,80* | *8,39* | *9,71* |
| *Trouvé* | *69,66* | *8,17* | *9,20* |

### Exemple 13 : Chlorhydrate de N-(4-{3-[(1R,5S)-3-azabicyclo[3.1.0]hex-3-yl]propoxy}phényl)-N-méthylacétamide

Le procédé expérimental est identique à celui de l'Exemple 1, voie de synthèse A, en remplaçant au stade 1 le 4-hydroxybenzamide par le *N*-(4-hydroxyphényl)-*N-*méthylacétamide.

### Microanalyse élémentaire :

| | *% C* | *% H* | *% N* | *% Cl* | *% Cl⁻* |
|---|---|---|---|---|---|
| *Calculé* | *62,86* | *7,76* | *8, 62* | *10,91* | *10,91* |
| *Trouvé* | *62,27* | *7,56* | *8,46* | *11,62* | *11,29* |

### Exemple 14 : N-(4-{3-[(1R,5S)-3-Azabicyclo[3.1.0]hex-3-yl]propoxy}phényl)-2-méthoxyacétamide

Le procédé expérimental est identique aux stades 1 et 2 de l'Exemple 1, voie de synthèse A, en remplaçant au stade 1 le 4-hydroxybenzamide par le *N*-(4-hydroxyphényl)-2-méthoxyacétamide.

### Microanalyse élémentaire :

| | *% C* | *% H* | *% N* |
|---|---|---|---|
| *Calculé* | *67,08* | *7,95* | *9,20* |
| *Trouvé* | *66,72* | *7,92* | *9,18* |

### Exemple 15 : N-(4-{2-[(1R,5S)-3-Azabicyclo[3.1.0]hex-3-yl]éthoxy}phényl)-2-méthoxyacétamide

Le procédé expérimental est identique aux stades 1 et 2 de l'Exemple 2 en remplaçant au stade 1 le 4-hydroxybenzamide par le *N*-(4-hydroxyphényl)-2-méthoxyacétamide.

### Microanalyse élémentaire :

| | *% C* | *% H* | *% N* |
|---|---|---|---|
| *Calculé* | *66,19* | *7, 64* | *9, 65* |
| *Trouvé* | *65,9* | *7,50* | *9,44* |

### Exemple 16 : Chlorhydrate de N-(4-{4-[(1R,5S)-3-azabicyclo[3.1.0]hex-3-yl]butoxy}phényl)-N-méthylacétamide

Le procédé expérimental est identique à celui de l'Exemple 13 en remplaçant au stade 1 le 1-bromo-3-chloropropane par le 1-bromo-4-chlorobutane.

### Microanalyse élémentaire :

| | *% C* | *% H* | *% N* | *% Cl* | *% Cl⁻* |
|---|---|---|---|---|---|
| *Calculé* | *65,47* | *8,52* | *7,63* | *9,66* | *9,66* |
| *Trouvé* | *65,35* | *8,41* | *7,30* | *9,80* | *9,67* |

### Exemple 17 : Chlorhydrate de N-(4-{4-[(1R,5S)-3-azabicyclo[3.1.0]hex-3-yl]butoxy}phényl)-2-méthoxyacétamide

Le procédé expérimental est identique à celui de l'Exemple 1, voie de synthèse A, en remplaçant au stade 1 le 4-hydroxybenzamide par le *N*-(4-hydroxyphényl)-2-méthoxyacétamide, et le 1-bromo-3-chloropropane par le 1-bromo-4-chlorobutane.

### Microanalyse élémentaire :

| | *% C* | *% H* | *% N* | *% Cl* | *% Cl⁻* |
|---|---|---|---|---|---|
| *Calculé* | *62,73* | *8,16* | *7,32* | *9,27* | *9,26* |
| *Trouvé* | *61,92* | *7,89* | *6,99* | *9,90* | *9,62* |

### Exemple 18 : 4-{3-[(1R,5S)-3-Azabicyclo[3.1.0]hex-3-yl]propoxy}benzamide

Le procédé expérimental reprend les stades 1 et 2 de l'Exemple 1, voie de synthèse A.

### Microanalyse élémentaire :

| | *% C* | *% H* | *% N* |
|---|---|---|---|
| *Calculé* | *69,21* | *7,74* | *10,76* |
| *Trouvé* | *69,24* | *7,69* | *10,60* |

### Exemple 19 : Oxalate de 4-{3-[(1R,5S)-3-azabicyclo[3.1.0]hex-3-yl]propoxy} benzamide

Le procédé expérimental reprend les stades 1 et 2 de l'Exemple 1, voie de synthèse A. Le composé ainsi obtenu est salifié suivant le mode opératoire décrit à l'Exemple 8.

### Microanalyse élémentaire :

| | *% C* | *% H* | *% N* |
|---|---|---|---|
| *Calculé* | *56,73* | *6,13* | *7,60* |
| *Trouvé* | *55,98* | *6, 28* | *6,94* |

### Exemple 20 : Citrate de 4-{3-[(1R,5S)-3-azabicyclo[3.1.0]hex-3-yl]propoxy}benzamide

On dissout un équivalent du composé de l'exemple 18 en présence d'1,2 équivalent d'acide citrique monohydraté dans l'eau pour conduire au produit du titre.

*RMN ¹H (600 MHz ; DMSO-d₆**)* : δ (ppm) = 9,5-12,5 (sl, 3H) ; 7,85 (m, 3H) ; 7,20 (sl, 1H) ; 6,95 (m, 2H) ; 4,05 (t, 2H) ; 3,30 (dl, 2H) ; 2,80-3 (2 sl, 4H) ; 2,65 (d, 2H) ; 2,55 (d, 2H) ; 1,95 (qt, 2H) ; 1,6 (sl, 2H) ; 0,5-0,7 (2 m, 2H).

### ETUDE PHARMACOLOGIQUE

### EXEMPLE A : Dosages cérébraux de la N^{τ}-Méthylhistamine chez la souris NMRI

Cette étude réalisée selon la méthode de Taylor et Coll. (Biochem. Pharm., 1992, 44, 1261-1267), a pour objectif d'évaluer l'activité *ex vivo* des composés de la présente invention en tant qu'antagonistes des récepteurs histaminergiques centraux de type H₃. Cette activité est révélée par la mesure, après traitement par voie orale des composés sous étude, des taux centraux de N^{τ}-Méthylhistamine, métabolite principal de l'histamine. Une augmentation des concentrations cérébrales de N^{τ}-Méthylhistamine indique une augmentation du turn-over de l'histamine par blocage des récepteurs histaminergiques centraux de type H₃.

Des souris NMRI (18-20g) sont traitées par voie orale par les composés de la présente invention ou par leur véhicule (20 ml/kg). Une heure après le traitement pharmacologique, les animaux sont sacrifiés, les cerveaux sont prélevés, congelés dans l'azote liquide, pesés et homogénéisés dans HClO₄ 0,1N à 4°C. Les homogénats sont centrifugés (15000g, 17 min, 4°C). Les surnageants sont récupérés et aliquotés. Les aliquotes sont congelés dans l'azote liquide et stockés à-80°C jusqu'à leur analyse. La détermination des taux cérébraux de N^{τ}-Méthylhistamine est réalisée par électrophorèse capillaire. Les taux tissulaires de N^{τ}-Méthylhistamine sont exprimés en µg/g de cerveau frais. La comparaison des taux cérébraux de N^{τ}-Méthylhistamine entre les animaux traités par le véhicule (témoins) et les animaux traités par les composés de la présente invention est effectuée par une analyse de variance à un facteur suivie si nécessaire par une analyse complémentaire (test de Dunnett).

Les résultats montrent que les composés de la présente invention sont capables, pour des doses comprises entre 1 et 30 mg/kg PO, d'augmenter de façon concentration-dépendante les concentrations cérébrales endogènes de N^{τ}-Méthylhistamine de plus de 200 %.

A titre d'exemple, les composés 1, 5, 6, 13 et 14 administrés à 10 mg/kg PO augmentent respectivement les concentrations cérébrales endogènes de N^{τ}-Méthylhistamine de 99 %, 109 %, 106 %, 135 % et 124 %, et de 227 %, 200 %, 210 %, 303 % et 266 %, à la dose de 30 mg/kg PO. Ces résultats indiquent que les composés de la présente invention sont de puissants antagonistes des récepteurs histaminergiques centraux de type H₃.

### EXEMPLE B : Enregistrements de l'électroencéphalogramme chez le rat Wistar vigile

Des rats Wistar mâles adultes sont implantés de manière chronique par des électrodes placées à la surface du cortex frontal et pariétal. Des enregistrements de l'électroencéphalogramme (EEG) cortical sont effectués chez les rats placés dans des cages dans une pièce isolée phoniquement. Les composés et véhicules sont administrés par voie intrapéritonéale de manière randomisée à 10h les mêmes jours avec un minimum de 3 jours entre chaque administration, permettant d'utiliser chaque rat comme son propre témoin. La puissance absolue des activités lentes delta (1-4 Hz), qui prédominent pendant le sommeil lent et disparaissent pendant l'éveil et le sommeil paradoxal, est moyennée sur des périodes successives de 30 min. Sur 30 min, des valeurs élevées et basses de la puissance des activités lentes delta sont des signes d'éveil et de sommeil, respectivement.
Les résultats montrent que les composés de la présente invention augmentent l'éveil (diminution des ondes delta) cortical chez le rat.
A titre d'exemple, les composés 1 et 13 administrés à la dose de 3 mg/kg IP, induisent une diminution significative de la puissance des ondes lentes delta pendant 120 minutes, signe d'activation cortical et d'éveil.

### EXEMPLE C : Interaction au barbital chez le rat Wistar

L'objectif de ce test est de déterminer les propriétés anti-sédatives, éveillantes et/ou anti-hypnotiques des composés de la présente invention. Les rats sont placés dans des cages individuelles et reçoivent une injection de barbital (170 mg/kg IP). La durée de sommeil est ensuite mesurée pendant 4 heures après l'injection de barbital. Elle est déterminée par la perte du réflexe de retournement. Les composés de l'invention ou leurs véhicules sont administrés par voie orale 30 minutes avant l'administration de barbital. Les résultats indiquent que les composés de la présente invention possèdent de puissantes activités anti-sédatives, anti-hypnotiques et/ou éveillantes.
Par exemple, à la dose de 30 mg/kg PO, les composés 5, 8, 12 et 15 diminuent la durée d'endormissement induite au barbital de -80%, -79%, -55% et -76%, respectivement.

### EXEMPLE D : Reconnaissance d'objet chez le Rat Sprague-Dawley

Le test de la reconnaissance d'objet chez le rat Sprague-Dawley (Behav. Brain Res., 1988, 31, 47-59) est basé sur l'activité exploratoire spontanée de l'animal et possède les caractéristiques de la mémoire épisodique chez l'homme. Sensible au vieillissement (Eur. J. Pharmacol., 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement par le véhicule par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté.
Par exemple, les résultats obtenus avec l'exemple 1 de la présente invention montrent une différence Delta de l'ordre de 6s à la dose de 0,3 mg/kg PO, ce qui indique que les composés de l'invention améliorent la mémorisation de façon importante, et à très faible dose.

### EXEMPLE E : Reconnaissance sociale chez le Rat Wistar

Initialement décrit en 1982 (J. Comp. Physiol., 1982, 96, 1000-1006), le test de la reconnaissance sociale a été ensuite proposé par différents auteurs (Psychopharmacology, 1987, 91, 363-368) ; Psychopharmacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester par voie intrapéritonéale et, 2 heures plus tard, est remis en présence (5 minutes) du jeune rat. Le comportement de reconnaissance sociale est alors à nouveau observé et la durée en est mesurée. Le critère de jugement est la différence (T2-T1), exprimée en secondes, des temps de « reconnaissance » des deux rencontres.
Les résultats obtenus avec l'exemple 1 montrent une différence (T2-T1) de 21s et de 26s pour des doses de 1 et 3 mg/kg IP, respectivement. Ceci montre que les composés de l'invention augmentent la mémorisation de façon très importante, et à faible dose.

### EXEMPLE F : Test d'enfouissement des billes chez la souris NMRI

Le comportement spontané d'enfouissement de billes chez la souris est un test empirique très sensible aux composés antidépresseurs (Sanchez et Meier, Psychopharmacology, 1997 ; 129 : 197-205 ; Nicolas et Prinssen, Eur. J. Pharmacol., 2006, 547 : 106-115). Les souris sont placées dans des cages individuelles dans lesquelles se trouvent 24 billes et le nombre de billes enfouies par chaque animal est déterminé pendant une période de 30 minutes. Les animaux sont traités par voie intrapéritonéale par les composés de la présente invention ou leurs véhicules 30 minutes avant de placer les animaux dans la cage remplie de billes.
Les résultats indiquent, par exemple, que les composés 1, 2, 5 et 15 à la dose de 30 mg/kg IP provoquent une diminution du nombre de billes enfouies de 48%, 99%, 49% et 83% respectivement.
Ce résultat indique que les composés de la présente invention possèdent des propriétés antidépressives.

### EXEMPLE G : Test de la suspension caudale chez la souris NMRI

Le test de suspension caudale chez la souris (Porsolt et al., Arch. Int. Pharmacodyn., 1987, 288, 11) permet la détection de propriétés psychopharmacologiques de composés. Des souris NMRI sont suspendues par la queue, à l'aide d'un morceau de ruban adhésif, à un crochet pendant 6 minutes : le temps d'immobilité est mesurée automatiquement à l'aide de capteurs de mouvements. Les animaux sont traités par voie intrapéritonéale par les composés de l'invention ou leurs véhicules, 24h et 30 minutes avant leur suspension.

Les résultats montrent que les exemples 1, 4, 5, 13, 14 et 15, administrés à la dose de 10 mg/kg IP, induisent une diminution du temps d'immobilité de 59%, 36%, 64%, 53%, 36% et 77%, respectivement. Ce résultat confirme les propriétés antidépressives des composés de la présente invention.

### EXEMPLE H : Test de la nage forcée chez la souris NMRI

Le test de la nage forcée est très largement utilisé pour détecter les propriétés antidépressives de composés (Porsolt et al., Arch. Int. Pharmacodyn., 1977, 229, 327-336). Les animaux sont placés dans des cylindres remplis d'eau (14 cm) pendant 6 minutes et la durée d'immobilité est mesurée pendant les 4 dernières minutes par un système de vidéotracking. Les composés de la présente invention ou leurs véhicules sont administrés par voie intrapéritonéale 30 minutes avant de placer les souris dans le cylindre.
Les exemples 1, 5 et 15 induisent à la dose de 30 mg/kg IP une diminution de l'immobilité de 76%, 32% et 34%, respectivement. Les résultats signent les propriétés antidépressives des composés de la présente invention.

### EXEMPLE I : Compositions pharmaceutiques

| Formule de préparation pour 1000 comprimés dosés à 100 mg : | |
|---|---|
| Composé de l'exemple 1 | 100 g |
| Hydroxypropylcellulose | 20 g |
| Polyvinylpyrrolidone | 20 g |
| Amidon de blé | 150 g |
| Lactose | 900 g |
| Stéarate de magnésium | 30 g |

## Revendications

1. Composés de formule (I) : 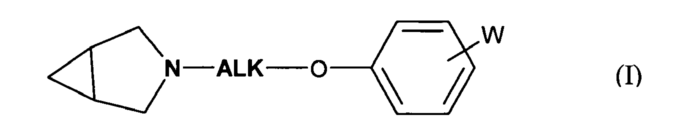 dans laquelle :
◆ Alk représente une chaîne alkylène,
◆ W représente un groupement choisi parmi 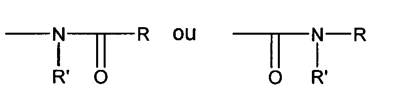 où R et R' représentent indépendamment l'un de l'autre un hydrogène ou un groupement alkyle (C₁-C₆) linéaire ou ramifié éventuellement substitué par un halogène, un groupement hydroxy ou un groupement alkoxy,
étant entendu que :
- le terme alkylène désigne un radical bivalent, linéaire ou ramifié, contenant de 2 à 6 atomes de carbone,
- le terme alkoxy désigne un groupement alkyle-oxy dont la chaîne alkyle, linéaire ou ramifiée, contient de 1 à 6 atomes de carbone,
leurs énantiomères et diastéréoisomères, ainsi que leurs sels d'addition à un acide ou à une base pharmaceutiquement acceptable.

2. Composés de formule (I) selon la revendication 1 pour lesquels le groupement W est situé en position para.

3. Composés de formule (I) selon la revendication 1 pour lesquels Alk représente un groupement éthylène, propylène ou butylène, leurs énantiomères et diastéréoisomères ainsi que leurs sels d'addition à un acide ou à une base pharmaceutiquement acceptables.

4. Composés de formule (I) selon la revendication 1 pour lesquels Alk représente un groupement propylène, leurs énantiomères et diastéréoisomères ainsi que leurs sels d'addition à un acide ou à une base pharmaceutiquement acceptables.

5. Composés de formule (I) selon la revendication 1 pour lesquels W représente un groupement -CO-NH₂, -NH-CO-CH₃, -N(CH₃)-CO-CH₃ ou -NH-CO-CH₂-OCH₃, leurs énantiomères et diastéréoisomères ainsi que leurs sels d'addition à un acide ou à une base pharmaceutiquement acceptables.

6. Composés de formule (I) selon la revendication 1 pour lesquels W représente un groupement -CO-NH₂, leurs énantiomères et diastéréoisomères ainsi que leurs sels d'addition à un acide ou à une base pharmaceutiquement acceptables.

7. Composés de formule (I) selon la revendication 1 qui sont :
- le *N*-(4-{3-[(1*R*,5*S*)-3-azabicyclo[3.1.0]hex-3-yl]propoxy}phényl)-*N-*méthylacétamide,
- le *N*-(4-{2-[(1*R*,5*S*)-3-azabicyclo[3.1.0]hex-3-yl]éthoxy}phényl)acétamide,
- le *N*-(4-{2-[(1*R*,5*S*)-3-azabicyclo[3.1.0]hex-3-yl]éthoxy}phényl)-2-méthoxyacétamide,
- le 4-{3-[(1*R*,5*S*)-3-azabicyclo[3.1.0]hex-3-yl]propoxy}benzamide,
- le 4-{2-[(1*R*,5*S*)-3-azabicyclo[3.1.0]hex-3-yl]éthoxy}benzamide,
- le *N*-(4-{3-[(1*R*,5*S*)-3-azabicyclo[3.1.0]hex-3-yl]propoxy}phényl)-2-méthoxyacétamide,
- le *N*-(4-{3-[(1*R*,5*S*)-3-azabicyclo[3.1.0]hex-3-yl]propoxy}phényl)acétamide,
et leurs sels d'adition à un acide ou à une base pharmaceutiquement acceptables.

8. Composé de formule (I) qui est le chlorhydrate de 4-{3-[(1*R*,5*S*)-3-azabicyclo[3.1.0]hex-3-yl]propoxy}benzamide.

9. Composé de formule (I) qui est l'oxalate de 4-{3-[(1*R*,5*S*)-3-azabicyclo[3.1.0]hex-3-yl]propoxy}benzamide.

10. Composé de formule (I) qui est le citrate de 4-{3-[(1*R*,5*S*)-3-azabicyclo[3.1.0]hex-3-yl]propoxy}benzamide.

11. Procédé de préparation des composés de formule (I) selon la revendication 1 **caractérisé en ce que** l'on utilise comme produit de départ un composé de formule (II) : 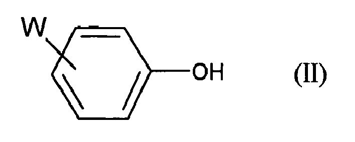 dans laquelle W est tel que défini dans la formule (I),
composé de formule (II) sur lequel on condense en milieu basique le composé de formule (III) :
Br-ALK-Cl (III)
pour obtenir le composé de formule (IV) : 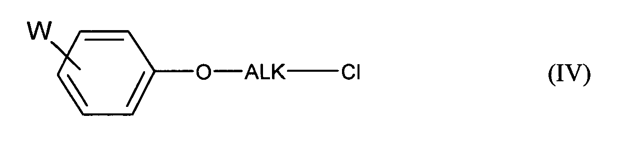 dans laquelle W et Alk sont tels que définis précédemment,
sur lequel on condense le composé de formule (V) : 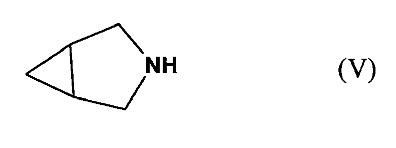 pour conduire au composé de formule (I) tel que défini précédemment : 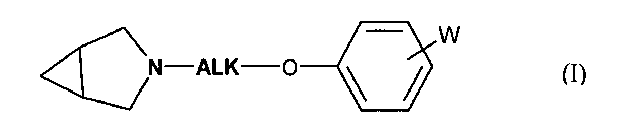 qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

12. Composés de formule (VI) suivante : 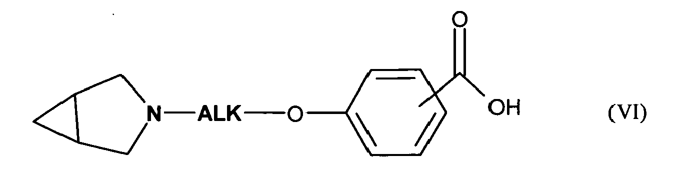 dans lesquels le groupement Alk est tel que défini dans la revendication 1,
utiles en tant qu'intermédiaires de synthèse des composés de formule (I) pour lesquels W=-CONRR'.

13. Composés de formule (VII) suivante : 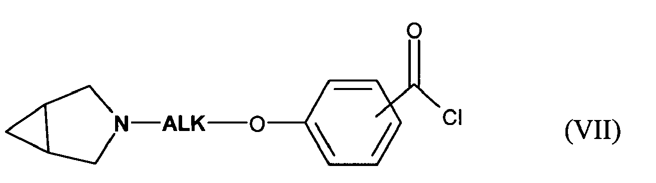 dans lesquels le groupement Alk est tel que défini dans la revendication 1,
utiles en tant qu'intermédiaires de synthèse des composés de formule (I) pour lesquels W=-CONRR'.

14. Composés de formule (VIII) suivante : 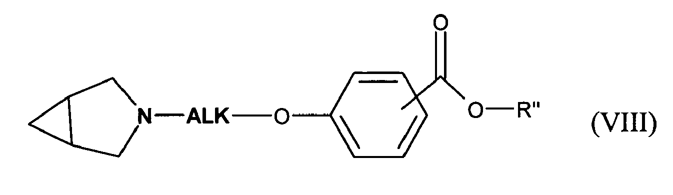 dans lesquels le groupement Alk est tel que défini dans la revendication 1 et R" est un groupe alkyle (C₁-C₆) linéaire ou ramifié,
utiles en tant qu'intermédiaires de synthèse des composés de formule (I) pour lesquels W=-CONRR'.

15. Composés de formule (IX) suivante : 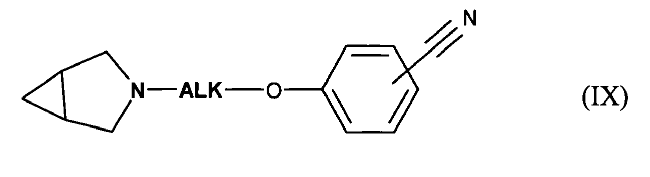 dans lesquels le groupement Alk est tel que défini dans la revendication 1,
utiles en tant qu'intermédiaires de synthèse des composés de formule (I) pour lesquels W=-CONRR'.

16. Compositions pharmaceutiques contenant comme principe actif au moins un composé de formule (I) selon l'une quelconque des revendications 1 à 10, ou un de ses sels d'addition à un acide ou à une base pharmaceutiquement acceptables, seul ou en combinaison avec un ou plusieurs excipients pharmaceutiquement acceptables.

17. Compositions pharmaceutiques selon la revendication 16 utiles pour le traitement des troubles cognitifs et psychocomportementaux associés au vieillissement cérébral, aux maladies neurodégénératives ou aux traumatismes crâniens, ainsi que dans le traitement des troubles de l'humeur, de la schizophrénie et des troubles cognitifs qui lui sont associés, des troubles du sommeil, du rythme veille-sommeil, du syndrome d'hyperactivité avec déficits attentionnels, ou de l'obésité.

18. Compositions pharmaceutiques selon la revendication 17 utiles pour le traitement des troubles cognitifs et psychocomportementaux associés à la maladie d'Alzheimer, à la maladie de Parkinson, à la maladie de Pick, aux démences à corps de Lewy, aux démences frontales et sous-corticales, aux démences frontotemporales, aux démences vasculaires, à la maladie de Huntington, ou à la sclérose en plaques.

19. Compositions pharmaceutiques selon la revendication 17 ou 18 utiles pour le traitement des troubles psychocomportementaux tels que les troubles du sommeil, l'apathie, et/ou les états dépressifs.

20. Compositions pharmaceutiques selon la revendication 17 utiles pour le traitement des troubles du sommeil tels que la narcolepsie, les hypersomnies survenant lors du syndrome d'apnée obstructive du sommeil ou du syndrome d'hyperactivité avec déficits attentionnels, ainsi que les somnolences diurnes.

21. Compositions pharmaceutiques selon la revendication 18 utiles pour le traitement des troubles du sommeil associés à la maladie d'Alzheimer et à la maladie de Parkinson.
